# EUROPEAN PATENT APPLICATION

(11) **EP 3 327 444 A1**
(43) Date of publication of application: **30.05.2018**
(21) Application number: 16830119.0
(22) Date of filing: 27.04.2016
(51) Int. Cl.: G01N 35/08, C12M 1/00, C12M 1/34, C12Q 1/68, G01N 21/78, G01N 33/50, G01N 33/58, G01N 37/00, C12N 15/09

(54) **KIT FOR ANALYSIS AND ANAYSIS METHOD USING SAME**

(30) Priority: 28.07.2015 JP 2015148961
(71) Applicant: Kabushiki Kaisha DNAFORM, Yokohama-shi Kanagawa 230-0046 (JP)
(72) Inventor: TSUJIMARU Koichiro, Yokohama-shi Kanagawa 230-0046 (JP)
(74) Representative: J A Kemp
(86) International application number: PCT/JP2016/063297
(87) International publication number: WO 2017/018014

(57) **Abstract**

The present invention provides a miniaturizable tool that can analyze a target in a sample by a simple operation and an analysis method using the tool. An analysis chip is a syringe-type chip including: a syringe main body having an openable and closable tip; and a piston to be inserted into the syringe main body, the piston being a hollow body having a translucent closed-end at a side to be inserted into the syringe main body and an open-end at the other side. Analysis device includes a main body case that is a housing, the housing including: an insertion opening into which the syringe-type chip is to be inserted; and a void in communication with the insertion opening, wherein an opposite end of the insertion opening in an axial direction is a bottom; a heating unit that heats the syringe-type chip; a light source unit that emits light to the syringe-type chip; and a detection unit that is a columnar body that can be inserted into the hollow body of the piston and disposed at the bottom of the housing in the axial direction. In use, the syringe-type chip is inserted into the analysis device from a side of the piston and the detection unit of the analysis device is inserted into the piston of the syringe-type chip.

## Description

### Technical Field

The present invention relates to an analysis kit and an analysis method using the same.

### Background Art

Nowadays, detection of the target gene of the source of infection in a biological sample is a common method for inspecting for infectious diseases due to viruses, bacteria, and the like. The detection of the target gene is commonly carried out by pretreating a collected biological sample, amplifying the nucleic acid of the target gene in the pretreated biological sample using a primer, and detecting the presence or absence or the amount of the nucleic acid amplification. The detection requires some steps, a special apparatus, and the like. Thus, the detection of the target gene is carried out in a medical institution such as a hospital or the like or in a special inspection agency.

On the other hand, the fact is that there is a demand for the detection not in an inspection agency or the like but at a personal level as described below. For example, when a patient has cold symptom, it is preferable if the patient can preliminarily test whether he/she has influenza also from the viewpoint of preventing secondary infection. Furthermore, among the infectious diseases, in particular, with reference to sexually transmitted diseases such as HIV, Chlamydia, and the like, a patient having such a disease often hesitates to take a test in a hospital and therefore tends to discover the disease too late. Thus, if a patient can take a test at home, such a disease can be discovered early.

For making the test at home implementable, an operation should be easy and a device for use in the test should be small, and attempts are being made to develop such a device. However, a small device with an easy operation that can be used at a personal level has not been provided so far because the test requires all of pretreatment of a biological sample, mixing of the biological sample and a reagent for nucleic acid amplification, amplification reaction of the nucleic acid, and detection of the reaction.

### Brief Summary of the Invention

### Problem to be Solved by the Invention

Hence, the present invention is intended to provide, for example, a miniaturizable tool that can analyze a target in a sample by a simple operation and an analysis method using the tool.

### Means for Solving Problem

In order to achieve the above object, the present invention provides a first analysis kit, including: an analysis chip that is a syringe-type chip, including: a syringe main body having an openable and closable tip; and a piston to be inserted into the syringe main body, the piston being a hollow body having a translucent closed-end at a side to be inserted into the syringe main body and an open-end at the other side; and an analysis device into which the analysis chip is to be inserted, including: a main body case that is a housing, the housing including: an insertion opening into which the syringe-type chip is to be inserted; and a void in communication with the insertion opening, wherein an opposite end of the insertion opening in an axial direction is a bottom; a heating unit that heats the syringe-type chip; a light source unit that emits light to the syringe-type chip; and a detection unit that is a columnar body that can be inserted into the hollow body of the piston and disposed at the bottom of the housing in the axial direction. In use, the syringe-type chip is inserted into the analysis device from a side of the piston and the detection unit of the analysis device is inserted into the piston of the syringe-type chip.

The present invention also provides a second analysis kit, including: an analysis chip that is a syringe-type chip, including: a syringe main body having an openable and closable tip and including a bead therein; and a piston to be inserted into the syringe main body; and an analysis device into which the analysis chip is to be inserted, including: a main body case that is a housing, the housing including: an insertion opening into which the syringe-type chip is to be inserted; and a void in communication with the insertion opening, wherein an opposite end of the insertion opening in an axial direction is a bottom; a heating unit that heats the syringe-type chip; a light source unit that emits light to the syringe-type chip; and a detection unit disposed at the bottom of the housing. In use, the syringe-type chip is inserted into the analysis device.

The present invention also provides a third analysis kit, including: an analysis chip that is a syringe-type chip, including: a syringe main body having an openable and closable tip; a sample chamber including an openable and closable sample inlet port and being connected to a tip region of the syringe main body; and a piston to be inserted into the syringe main body; and an analysis device into which the analysis chip is to be inserted, including: a main body case that is a housing, the housing including: an insertion opening into which the syringe-type chip is to be inserted; and a void in communication with the insertion opening, wherein an opposite end of the insertion opening in an axial direction is a bottom; a heating unit that heats the syringe-type chip; a light source unit that emits light to the syringe-type chip; and a detection unit disposed at the bottom of the housing. In use, the syringe-type chip is inserted into the analysis device.

The present invention also provides an analysis method of a sample using the analysis kit according to the present invention, including the following steps: introducing a sample into the syringe-type chip; mixing the sample and a reagent in the syringe-type chip; inserting the syringe-type chip into the analysis device; causing the sample to thermally react with the reagent in the syringe-type chip by the heating unit of the analysis device; and detecting the reaction in the syringe-type chip by the detection unit of the analysis device.

### Effects of the Invention

According to the present invention, for example, an analysis chip and an analysis device can be downsized. Furthermore, the present invention allows, for example, an analyst to analyze a target in a sample easily by preliminarily mixing a sample and a reagent using the analysis chip to prepare a reaction system and then simply inserting the analysis chip into the analysis device. Thus, for example, viral infection due to an influenza virus and the like and sexually transmitted diseases such as HIV, chlamydia, and the like can be analyzed easily. In particular, the present invention allows an analyst to analyze a target easily by himself/herself without going to an inspection agency such as a hospital or the like, for example, because the analysis device can be downsized.

### Brief Description of Drawings

[FIG. 1] FIG. 1A is a top view schematically showing an example of an analysis chip in an analysis kit of the present invention; and FIG. 1B is a cross sectional view of the analysis chip shown in FIG. 1A.
[FIG. 2] FIG. 2 is a cross sectional view schematically showing an example of an analysis device in the analysis kit of the present invention.
[FIG. 3] FIG. 3 shows cross sectional views schematically showing an example of the usage of the analysis kit of the present invention.

### Mode for Carrying out the Invention

The present invention is described in more detail below with reference to examples. The present invention, however, is not limited by the following description.

The present invention relates to an analysis kit and an analysis method using the same. According to the present invention, for example, the analysis method of the present invention can be performed by mixing a sample and a reagent to prepare a reaction system using the analysis chip and then simply setting the analysis chip in the analysis device.

Though the analysis kit of the present invention is eventually set in the analysis device in use, the analysis chip and the analysis device may be independent from each other when the analysis kit is not in use.

The analysis kit of the present invention includes the above-described first, second, and third analysis kits. The first, second, and third analysis kits of the present invention can be used in any combination, for example. Specifically, examples of the combination include the combination of the first analysis kit and second analysis kit of the present invention, the combination of the first analysis kit, second analysis kit, and third analysis kit of the present invention, and the combination of the second analysis kit and third analysis kit of the present invention. These analysis kit will be described in detail below. The descriptions as to the first, second, and third analysis kits of the present invention can be referred to one another, for example, unless otherwise noted.

### [First analysis kit and analysis method]

As described above, the first analysis kit of the present invention is an analysis kit, including: an analysis chip that is a syringe-type chip, including: a syringe main body having an openable and closable tip; and a piston to be inserted into the syringe main body, the piston being a hollow body having a translucent closed-end at a side to be inserted into the syringe main body and an open-end at the other side; and an analysis device into which the analysis chip is to be inserted, including: a main body case that is a housing, the housing including: an insertion opening into which the syringe-type chip is to be inserted; and a void in communication with the insertion opening, wherein an opposite end of the insertion opening in an axial direction is a bottom; a heating unit that heats the syringe-type chip; a light source unit that emits light to the syringe-type chip; and a detection unit that is a columnar body that can be inserted into the hollow body of the piston and disposed at the bottom of the housing in the axial direction. In use, the syringe-type chip is inserted into the analysis device from a side of the piston and the detection unit of the analysis device is inserted into the piston of the syringe-type chip.

In the first analysis kit, the piston of the syringe-type chip is a hollow body and the detection unit of the analysis device is a columnar body that can be inserted into the hollow body. Thus, the detection unit of the analysis device can be inserted into the piston when the syringe-type chip is inserted into the analysis device. Since the piston has a translucent closed-end, the reaction of a sample in the syringe main body can be detected by the detection unit through the closed-end of the piston.

In the first analysis kit, it is only required that the closed-end of the piston is a translucent end. For example, the closed-end is formed of a transparent member.

In the first analysis kit, for example, the detection unit includes a support and a detector. The support is a columnar body, and the detector is disposed at a tip of the support. The detector can be, for example, an optical sensor such as CCD, avalanche photodiode (APD), or the like.

The first analysis method of the present invention is an analysis method of a sample using the first analysis kit, including the following steps: introducing a sample into the syringe-type chip; mixing the sample and a reagent in the syringe-type chip; inserting the syringe-type chip into the analysis device; causing the sample to thermally react with the reagent in the syringe-type chip by the heating unit of the analysis device; and detecting the reaction in the syringe-type chip by the detection unit of the analysis device. In the insertion step, the syringe-type chip is inserted into the analysis device and the detection unit of the analysis device is inserted into the piston of the syringe-type chip.

### [Second analysis kit and analysis method]

As described above, the second analysis kit of the present invention is an analysis kit, including: an analysis chip that is a syringe-type chip, including: a syringe main body having an openable and closable tip and including a bead therein; and a piston to be inserted into the syringe main body; and an analysis device into which the analysis chip is to be inserted, including: a main body case that is a housing, the housing including: an insertion opening into which the syringe-type chip is to be inserted; and a void in communication with the insertion opening, wherein an opposite end of the insertion opening in an axial direction is a bottom; a heating unit that heats the syringe-type chip; a light source unit that emits light to the syringe-type chip; and a detection unit disposed at the bottom of the housing. In use, the syringe-type chip is inserted into the analysis device.

In the second analysis kit, the syringe main body includes the bead therein. The bead allows a sample and a reagent to be mixed easily in the syringe main body, for example.

In the second analysis kit, preferably, the bead is a magnetic bead, for example. The magnetic bead can be, for example, a metal bead.

In the second analysis kit, when the bead is the magnetic bead, preferably, the analysis device further includes a magnet, for example. The magnet is preferably disposed on the inner surface of the void corresponding to a transmission limit region of the magnetic bead in the syringe main body in the state where the syringe-type chip is inserted in the analysis device, for example. In this case, for example, by inserting the analysis chip into the analysis device after the mixing using the magnetic bead, the magnetic bead of the syringe main body can be captured by the magnet.

In the case where a sample is analyzed optically using the analysis kit of the present invention, the length of the syringe main body in the axial direction is a light path length, and securing this light path length is important when a sample is introduced. The capture of the magnetic bead by the magnet of the analysis device prevents the magnetic bead from being suspended and secures the light path length, and this allows the detection with higher accuracy.

The magnet is not limited as long as it can capture the magnetic bead, and can be a neodymium magnet, for example.

In the second analysis kit, preferably, the syringe-type chip further includes a negative pressure generator, and the negative pressure generator is connected to the tip region of the syringe main body, for example. In this case, for example, after a sample has introduced into the syringe main body and mixed using the bead, a negative pressure is generated in the syringe main body by the negative pressure generator. The bead in the syringe main body is attracted to the tip region of the syringe main body by the negative pressure and captured by the negative pressure at a site where the bead cannot pass through. This prevents the bead from being suspended and avoids the influence of the suspended bead when the inside of the syringe main body is detected by the detection unit. Furthermore, when the bead is a magnetic bead, for example, the capture of the magnetic bead by the magnet of the analysis device and by the negative pressure allows the magnetic bead to be captured more securely.

The second analysis method of the present invention is an analysis method of a sample using the second analysis kit, including the following steps: introducing a sample into the syringe-type chip; mixing the sample and a reagent in the syringe-type chip; inserting the syringe-type chip into the analysis device; causing the sample to thermally react with the reagent in the syringe-type chip by the heating unit of the analysis device; and detecting the reaction in the syringe-type chip by the detection unit of the analysis device. In the mixing step, the sample and the reagent are mixed using the bead of the syringe-type chip.

In the second analysis method, preferably, the bead is a magnetic bead, and, in the insertion step, the syringe-type chip is inserted into the analysis device, and the magnetic bead of the syringe-type chip is captured by the magnet of the analysis device, for example.

In the second analysis method, for example, after the insertion step and before the reaction step, a negative pressure is generated in the syringe main body by a negative pressure generator of the syringe-type chip.

### [Third analysis kit and analysis method]

As described above, the third analysis kit of the present invention is an analysis kit, including: an analysis chip that is a syringe-type chip, including: a syringe main body having an openable and closable tip; a sample chamber including an openable and closable sample inlet port and being connected to a tip region of the syringe main body; and a piston to be inserted into the syringe main body; and an analysis device into which the analysis chip is to be inserted, including: a main body case that is a housing, the housing including: an insertion opening into which the syringe-type chip is to be inserted; and a void in communication with the insertion opening, wherein an opposite end of the insertion opening in an axial direction is a bottom; a heating unit that heats the syringe-type chip; a light source unit that emits light to the syringe-type chip; and a detection unit disposed at the bottom of the housing. In use, the syringe-type chip is inserted into the analysis device.

In the third analysis kit, the analysis chip includes the sample chamber. Thus, a sample can be introduced into the sample chamber first and then introduced into the syringe main body by the piston, and this allows an easy operation, for example. In addition, the sample can be introduced into the syringe main body by closing the sample inlet port after the introduction of the sample into the sample chamber, and this improves operational safety. In particular, it is effective in the case of using a biological sample such as blood, saliva, or the like which raises concern about infection and the like.

In the third analysis kit, preferably, the syringe-type chip further includes a negative pressure generator, the negative pressure generator is connected to the tip region of the syringe main body, and the connection site of the negative pressure generator and the tip region is located closer to the tip of the syringe main body in comparison with a connection site of the tip region and the sample chamber, for example. In this case, preferably, after a sample has introduced into the syringe main body, a negative pressure is generated in the syringe main body by the negative pressure generator, for example. The negative pressure allows the flow channel between the syringe main body and the chamber and the flow channel between the chamber and the negative pressure generator to be filled with the sample to a sufficient degree and is effective for securing the light path length.

In the third analysis kit, for example, at least one of the flow channel between the syringe main body and the chamber and the flow channel between the chamber and the negative pressure generator may preliminarily be filled with a solvent. By preliminarily filling the flow channel with a solvent, for example, the flow channel between the syringe main body and the chamber and the flow channel between the chamber and the negative pressure generator can be filled with a liquid entirely when the sample is introduced from the sample chamber.

The third analysis method of the present invention is an analysis method of a sample using the third analysis kit, including the following steps: introducing a sample into the syringe-type chip; mixing the sample and a reagent in the syringe-type chip; inserting the syringe-type chip into the analysis device; causing the sample to thermally react with the reagent in the syringe-type chip by the heating unit of the analysis device; and detecting the reaction in the syringe-type chip by the detection unit of the analysis device. In the introduction step, after the sample has introduced into the sample chamber of the syringe-type chip through the sample inlet port, the sample is introduced into the syringe main body from the sample chamber.

In the third analysis method, preferably, after the introduction step and before the reaction step, a negative pressure is generated in the syringe main body by a negative pressure generator of the syringe-type chip, for example.

In the analysis kit of the present invention, for example, the first analysis kit may further satisfy the requirements of the second analysis kit and may further satisfy the requirements of the third analysis kit.

In the analysis kit of the present invention, for example, the second analysis kit may further satisfy the requirements of the third analysis kit.

In the analysis kit of the present invention, the material of the main body case is not limited to a particular material, and can be, for example, a plastic member.

In the analysis kit of the present invention, the heating unit is not limited to a particular type, and the heating unit can be any heating unit as long as it can heat the syringe-type chip to be inserted. For example, a heater can be used as the heating unit.

In the analysis kit of the present invention, preferably, the heating unit and the light source unit are respectively disposed on the inner surfaces of the void along the axial direction, for example.

In the analysis kit of the present invention, preferably, the heating unit and the light source unit each are disposed on each of the inner surfaces of the void facing each other, for example.

In the analysis kit of the present invention, preferably, the analysis device further includes a heat insulation section, and the heating unit is disposed on the inner surface of the void through the heat insulation section, for example. In the analysis device, for example, the heat insulation section may be disposed only in a region where the heating unit is disposed. For example, the heat insulation section may be disposed also outside the region where the heating unit is disposed because it allows sufficient heat insulation performance. As a specific example, the heat insulation section may be disposed in the whole inner region of the main body case. The heat insulation section is not limited to a particular type, and a common heat insulation material can be used.

In the analysis kit of the present invention, preferably, the light source unit includes: a linear light source; and a converter that converts linear light into surface light. The linear light source emits linear light along the axial direction of the reaction flow channel, and the converter converts the linear light into surface light and emits the surface light, for example. The converter is not limited to a particular converter and can be, for example, a light guide plate or the like.

In the void of the main body case of the device, for example, the light source unit may be disposed either above or below the syringe-type chip or may be disposed both above and below the syringe-type chip.

There is no particular limitation on the type of the light emitted from the light source unit. Also, the light source unit is not limited to a particular type, and an LED, an optical fiber, and the like can be used, for example. The type of the light source unit can be determined appropriately according to the type of the reagent for the target, for example. Preferably, the light source unit can emit excitation light suitable for the reagent. The light may be emitted from the light source unit as follows, for example. That is, a white light source may be used as a light source and desired excitation light may be obtained through a polarizing filter to emit the obtained excitation light.

In the analysis kit of the present invention, the detection unit is not limited to a particular unit, and examples thereof include optical sensors such as CCD, APD, and the like. Preferably, the detection unit can detect luminescence, fluorescence, and the like that are generated by the direct or indirect reaction of a reagent and a target in a sample, for example.

In the analysis kit of the present invention, for example, the piston includes an optical fiber, preferably.

In the analysis kit of the present invention, for example, the syringe-type chip may include at least one of a condenser lens and an optical fiber therein. Such a configuration allows the detection of the reaction in accordance with the distance in the syringe main body of the syringe-type chip, for example. Thus, the position where the reaction is caused in the syringe main body can be analyzed. This allows scanning in the syringe main body and also allows counting of the target in the sample, for example.

The analysis kit of the present invention further includes: a power supply; or a connector to be connected to an external power supply, or the like, for example. Examples of the external power supply include a personal computer, a tablet, and a cellular phone. The connector can be, for example, a USB connector and the like.

In the analysis kit of the present invention, preferably, the housing includes a guide through which the syringe-type chip is introduced into the housing.

The analysis kit of the present invention is for nucleic acid analysis, for example.

In the analysis kit of the present invention, preferably, the syringe-type chip includes a reagent therein, for example. In the syringe-type chip, the reagent may be disposed in a dry form or in a liquid form, for example. In the former case, for example, by introducing a liquid sample into the syringe-type chip as the sample, the reagent in a dry form and the sample may be mixed, or, by adding a solvent to the syringe-type chip, the sample and the reagent may be mixed. The solvent is not limited to a particular solvent, and examples of the solvent include water, a buffer solution, physiological saline, and mixtures thereof.

The reagent includes an analysis reagent that reacts with a target in a sample, for example. When the target is a nucleic acid, the analysis reagent includes at least one of a reagent for nucleic acid amplification and a reagent for nucleic acid detection, for example. The reagent for nucleic acid detection can be, for example, a fluorescence reagent. The reagent may include a pretreatment reagent of a sample, for example.

Examples of the reagent for nucleic acid amplification include a primer, enzyme, and components that can be used for nucleic acid amplification. The primer can be determined appropriately according to the type of the target, for example. The primer can be, for example, a labeled primer. As the labeled primer, for example, a primer that has an exciton effect described in Japanese Patent No. 4370385 and the like can be used. The enzyme can be, for example, an enzyme for nucleic acid amplification and examples thereof include polymerase such as DNA polymerase, RNA polymerase, or the like; and reverse transcriptase.

The reagent for nucleic acid detection can be, for example, a probe and the like to a target. The probe may be labeled with a labeling substance, for example. The labeling substance is not limited to a particular substance, and the labeling substance can be, for example, a fluorescent material. As the labeled probe, for example, a probe that has an exciton effect described in Japanese Patent No. 4761086 and the like can be used. Also, as the reagent for nucleic acid detection, for example, an intercalator such as SYBR (registered trademark) Green or the like and a ruthenium complex can be used. The reagent for nucleic acid detection can be, for example, a fluorescence reagent.

In the analysis kit of the present invention, for example, the analysis chip may be a connected syringe-type chip in which plural syringe-type chips are connected in parallel, and the analysis device may be a multi-analysis device including a plurality of detection units corresponding to the plural syringe-type chips.

In the analysis kit of the present invention, for example, in a state where the connected syringe-type chip is inserted in the analysis device, the heating unit is disposed on the inner surface of the void so as to face a parallel surface of each syringe-type chip of the connected syringe-type chip, and the light source unit is disposed at least above or below the syringe-type chip at at least one end of the connected syringe-type chip and extended along the axial direction of the connected syringe-type chip.

In the analysis kit of the present invention, preferably, the light source unit includes a linear light source; and a converter that converts linear light into surface light. The linear light source emits linear light along the axial direction of the connected syringe-type chip, and the converter converts the linear light into surface light and emits the surface light, for example. Since the linear light emitted along the axial direction of the syringe-type chip is converted into surface light, when the analysis chip is inserted in the analysis device of the present invention, the parallel surface of the syringe-type chip can be irradiated with the surface light. In other words, in the analysis device, although the linear light source is disposed in the vicinity of the syringe-type chip at an end of the connected syringe-type chip, each syringe-type chip of connected syringe-type chip can be entirely irradiated with light more effectively, for example.

For example, in the void of the main body case of the device, the light source unit may be disposed only above the syringe-type chip at one end or the other end of the connected syringe-type chip, the light source unit may be disposed only below the syringe-type chip at one end or the other end of the connected syringe-type chip, the light source units may be disposed only above or below the syringe-type chips at both ends of the connected syringe-type chip of the analysis chip, or the light source units may be disposed above the syringe-type chip at one end of the connected syringe-type chip and below the syringe-type chip at the other end of the connected syringe-type chip. When the light source units are disposed at the syringe-type chips at both ends of the connected syringe-type chip, preferably, the light source units are disposed such that the direction of the linear light emitted from one light source unit is in the opposite direction to the direction of the linear light emitted from the other light source unit.

In the analysis kit of the present invention, for example, the parallel surface of each syringe-type chip of the connected syringe-type chip is a translucent parallel surface, and a side surface of each syringe-type chip that is in contact with an adjacent syringe-type chip in the connected syringe-type chip has light blocking ability. In the case of the connected syringe-type chip, the reaction in each syringe-type chip is detected by each of the detection units of the analysis device. Thus, for example, there is a possibility that the detection unit detects not only the reaction of a corresponding syringe-type chip but also the reaction of an adjacent syringe-type chip. However, according to the above described configuration, since the side surface has light blocking ability, the reaction of an adjacent syringe-type chip can be prevented from being detected by the detection units, whereby the detection accuracy can further be increased.

In the analysis kit of the present invention, for example, at least two syringe-type chips of the connected syringe-type chip include analysis reagents that react with different targets in a sample. This allows an analyst to analyze different targets in the syringe-type chips with a single analysis kit, for example.

In the analysis method of the present invention, the analysis target is not limited to a particular target. The target can be, for example, a nucleic acid. The nucleic acid is not limited to a particular type, and examples thereof include DNA, cDNA, and RNA. The RNA can be, for example, mRNA and miRNA. The origin of the nucleic acid is not particularly limited, and examples thereof include viruses, bacteria, and mold. Examples of the virus include various influenza viruses, HIV, and herpes. Examples of the bacterium include Chlamydia, Neisseria gonorrhoeae, and Treponema (syphilis). Examples of the mold include fungi such as Candida and the like.

In the analysis method of the present invention, the sample is not limited to a particular sample, and examples of the sample include animal-derived samples; plant-derived samples; environmental samples such as seawater, soil, drainage, and the like; and food and beverage samples such as drinking water, food, and the like. Examples of the animal-derived sample include biological samples such as blood (including whole blood and isolated blood cell); blood serum; blood plasma; cells (including cultured cells); tissues; body fluid (e.g., ear discharge, nasal discharge, pus, ascites, pleural fluid, bile, spinal fluid, expectoration, and the like); mucosa cells (e.g., oral mucosa cells, gastric mucosa cells, respiratory mucosa, and the like); swabs collected from nasal mucosa, oral mucosa, and the like using a cotton swab or the like; sweat; amniotic fluid; excreta (e.g., urine, feces, and the like); brushing collected from organs using an endoscope or the like; collected liquid; biopsy samples; alveolar washing liquid; and the like. In the analysis method of the present invention, preferably, the sample to be introduced into the reaction system preparation chip is a pretreated sample, for example.

In the analysis method of the present invention, the nucleic acid amplification reaction can be performed by using the sample and the reagent. The nucleic acid amplification reaction is not limited to a particular type, and examples of the nucleic acid amplification reaction include the PCR method, TMA method, NASBA method, LAMP method, ICAN method, RCA method, SDA method, HDA method, and SmartAmp method. Among them, an isothermal amplification method such as the SmartAmp method is preferable because it allows treatment at a constant temperature.

Examples of the analysis chip and the analysis method of the present invention are described with reference to the drawings. Although the following example is an example of the combination of the first, second, and third analysis kits, the analysis chip and the analysis method of the present invention are not limited thereto.

In the drawings, identical parts are indicated with identical numerals and symbols. Also, the drawings are schematic views, and the size and shape of components are not limited thereto.

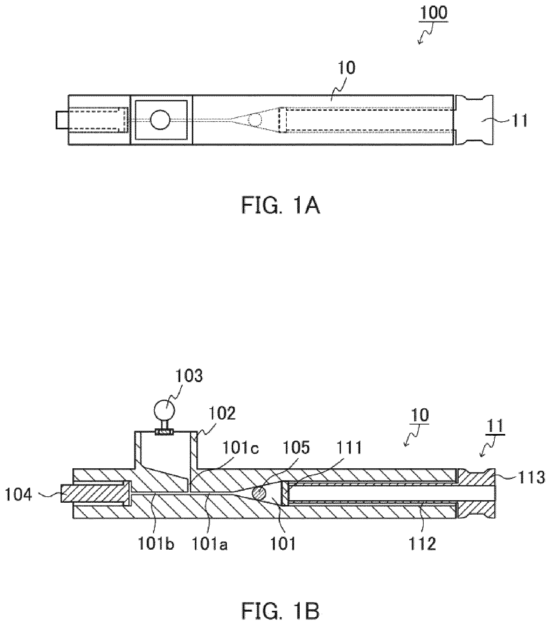

FIG. 1A is a top view schematically showing an example of an analysis chip of the present invention; and FIG. 1B is an axial cross sectional view of the analysis chip shown in FIG. 1A. FIGs. 1A and 1B show the combination of the analysis chip of the first analysis kit, the analysis chip of the second analysis kit, and the analysis chip of the third analysis kit, i.e., show an analysis chip having the configurations of these.

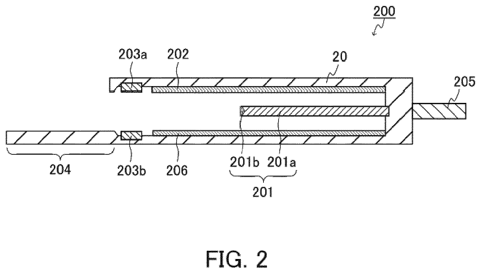

FIG. 2 is a cross sectional view schematically showing an example of an analysis device of the present invention. FIG. 2 shows the combination of the analysis device of the first analysis kit, the analysis device of the second analysis kit, and the analysis device of the third analysis kit, i.e., shows an analysis device having the configurations of these.

As shown in FIGs. 1A and 1B, a syringe-type chip 100 which is an analysis chip includes a syringe main body 10 and a piston 11. The syringe main body 10 includes a void 101 which is tapered toward the tip, a magnetic bead 105 is disposed in the void 101. A flow channel 101a of the syringe main body 10 at a tip region is in communication with a negative pressure generator 104 through a flow channel 101b and is in communication with a sample chamber 102 through a flow channel 101c. In the present example, the negative pressure generator 104 is a piston inserted in the void in communication with the flow channel 101c. The sample chamber 102 includes a sample inlet port and a cap 103 at the upper part thereof. The cap 103 allows the sample inlet port to be opened and closed. The piston 11 has a hollow body 112. The piston 11 includes a closed end 111 at the side to be inserted into the syringe main body 10 and an open-end at the other side. The piston 11 further includes a gripping member 113 at the same side as the open-end. The end 111 is translucent. The piston 11 is inserted in the void 101 of the syringe main body 10 before use. By pulling the gripping member 113, a negative pressure can be generated in the syringe main body.

As shown in FIG. 2, an analysis device 200 includes a housing 20 that is a main body case and a connector 205. The housing 20 includes an insertion opening into which the syringe-type chip 100 is to be inserted and a void. In the void, a detection unit 201 is disposed along the axial direction, a light source unit 202 is disposed at the upper surface side, and a heating unit 206 is disposed at the lower surface side. The housing 20 includes a guide region 204 at the upstream of the insertion opening for helping the insertion of the syringe-type chip 100. The analysis device 200 further includes a pair of magnets on the upper inner surface and the lower inner surface of the housing 20 for capturing a magnetic bead 105 disposed in the syringe-type chip 100 when the syringe-type chip 100 is inserted into the analysis device 200. In the present example, the connector 205 is a USB connector. The detection unit 201 includes a support 201a and a CCD 201b, and the CCD 201b is attached to the tip of the support 201a.

There is no particular limitation on the size of the analysis device 200. The thickness of the analysis device 200 can be, for example, 10 mm ± 5 mm or about 8 mm. There are no particular limitations on the length of the analysis device 200 in the longitudinal direction (the length of the syringe-type chip 100 in the insertion direction) and the length of the analysis device 200 in the width direction (the vertical direction to the insertion direction).

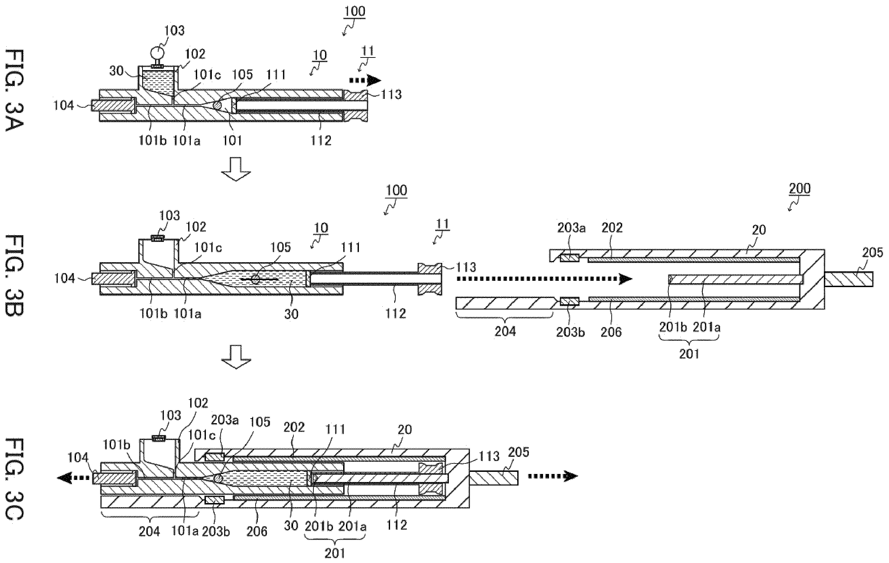

The analysis method of a sample using the syringe-type chip 100 and the analysis device 200 is described below with reference to FIG. 3.

In the void 101 of the syringe main body 10, a reagent is preliminarily disposed in accordance with a desired target. As to the syringe-type chip 100, the cap 103 of the sample chamber 102 is uncapped, the piston 11 is inserted into the void 101 of the syringe main body 10 as far as possible, and the negative pressure generator 104 is pushed into the void which is in communication with the flow channel 101c of the syringe main body 10. Next, a sample 30 is introduced into the sample chamber 102 through the sample inlet port and the sample inlet port is closed with the cap 103. Then, as shown in (A) in FIG. 3, the piston 11 is pulled in the direction of the arrow. This generates a negative pressure in the void 101 of the syringe main body 10, and the sample 30 in the sample chamber 102 is introduced into the void 101 of the syringe main body 10 through the flow channel 101c and the flow channel 101a.

Next, as shown in (B) in FIG. 3, the syringe-type chip 100 is shaken in the axial direction of the syringe main body 10 so as to move the magnetic bead 105 in the void 101, whereby the sample 30 and the reagent in the void 101 are mixed. On this occasion, bubbles in the void 101 can be removed by the movement of the magnetic bead 105.

Then, as shown in (B) in FIG. 3, the syringe-type chip 100 is moved in the direction of the arrow and inserted into the void through the guide region 204 of the analysis device 200.

On this occasion, as shown in (C) of FIG. 3, the detection unit 201 of the analysis device 200 is inserted into the hollow body 112 of the piston 11 of the syringe-type chip 100, and the magnetic bead 105 of the syringe-type chip 100 is captured by a pair of magnets 203a and 203b of the analysis device 200. The sites where the magnets 203a and 203b are disposed correspond to a site having an opening size where the magnetic bead 105 cannot pass through in the void 101 of the syringe-type chip 100 inserted in the analysis device 200. This secures the light path length in the syringe main body 10.

Next, as shown in (C) in FIG. 3, the piston that is the negative pressure generator 104 of the syringe-type chip 100 is pulled in the direction of the arrow on the left. Thereby, a negative pressure is generated in the flow channel 101b of the syringe main body 10, and the magnetic bead 105 captured by the magnets 105a and 105b is attracted in the direction of the arrow on the left and fixed there.

Thereafter, the reaction solution of the sample 30 and the reagent in the void 101 of the syringe main body 10 is heated by the heating unit 206, and then the light is emitted from the light source unit 202. Then, the reaction solution is detected by the CCD 201b at the tip of the detection unit 201 of the analysis device 200 through the translucent end 111 of the piston 11.

This application claims priority from Japanese Patent Application No. 2015-148961filed on July 28, 2015. The entire subject matter of the Japanese Patent Application is incorporated herein by reference.

### Industrial Applicability

According to the present invention, for example, an analysis chip and an analysis device can be downsized. Furthermore, the present invention allows, for example, an analyst to analyze a target in a sample easily by preliminarily mixing a sample and a reagent using the analysis chip of the present invention to prepare a reaction system and simply inserting the analysis chip into the analysis device. Thus, for example, viral infection due to an influenza virus and the like and sexually transmitted diseases such as HIV, Chlamydia, and the like can be analyzed easily. In particular, the present invention allows an analyst to analyze a target easily by himself/herself without going to an inspection agency such as a hospital or the like, for example, because the analysis device can be downsized.

### Explanation of Reference Numerals

- 100: syringe-type chip
- 10: syringe main body
- 101a, 101b, 101c: flow channel
- 102: sample chamber
- 103: cap
- 104: negative pressure generator
- 105: magnetic bead
- 11: piston
- 111: translucent end
- 112: hollow body
- 113: gripping member
- 200: analysis device
- 20: housing
- 201: detection unit
- 201a: support
- 201b: CCD
- 202: light source unit
- 203a, 203b: magnet
- 204: guide region
- 205: connector
- 206: heating unit
- 30: sample

## Claims

1. An analysis kit, comprising:
an analysis chip that is a syringe-type chip, comprising:
a syringe main body having an openable and closable tip; and
a piston to be inserted into the syringe main body, the piston being a hollow body having a translucent closed-end at a side to be inserted into the syringe main body and an open-end at the other side; and
an analysis device into which the analysis chip is to be inserted, comprising:
a main body case that is a housing, the housing
comprising:
an insertion opening into which the syringe-type chip is to be inserted; and
a void in communication with the insertion opening, wherein
an opposite end of the insertion opening in an axial direction is a bottom;
a heating unit that heats the syringe-type chip;
a light source unit that emits light to the syringe-type chip; and
a detection unit that is a columnar body that can be inserted into the hollow body of the piston and disposed at the bottom of the housing in the axial direction, wherein
in use, the syringe-type chip is inserted into the analysis device from a side of the piston and the detection unit of the analysis device is inserted into the piston of the syringe-type chip.

2. The analysis kit according to claim 1, wherein
the detection unit comprises a support and a detector,
the support is a columnar body, and
the detector is disposed at a tip of the support.

3. The analysis kit according to claim 1 or 2, wherein
the detector is at least one of CCD and APD.

4. The analysis kit according to any one of claims 1 to 3, wherein the closed-end of the piston is formed of a transparent member.

5. An analysis kit, comprising:
an analysis chip that is a syringe-type chip, comprising:
a syringe main body having an openable and closable tip and comprising a bead therein; and
a piston to be inserted into the syringe main body; and
an analysis device into which the analysis chip is to be inserted, comprising:
a main body case that is a housing, the housing
comprising:
an insertion opening into which the syringe-type chip is to be inserted; and
a void in communication with the insertion opening, wherein
an opposite end of the insertion opening in an axial direction is a bottom;
a heating unit that heats the syringe-type chip;
a light source unit that emits light to the syringe-type chip; and
a detection unit disposed at the bottom of the housing, wherein
in use, the syringe-type chip is inserted into the analysis device.

6. The analysis kit according to claim 5, wherein
the bead is a magnetic bead.

7. The analysis kit according to claim 6, wherein
the analysis device further comprises a magnet, and
the magnet is disposed on an inner surface of the void corresponding to a transmission limit region of the magnetic bead in the syringe main body in a state where the syringe-type chip is inserted in the analysis device.

8. The analysis kit according to claim 7, wherein
the magnet is a neodymium magnet.

9. The analysis kit according to any one of claims 5 to 8, wherein
the syringe-type chip further comprises a negative pressure generator, and
the negative pressure generator is connected to a tip region of the syringe main body.

10. An analysis kit, comprising:
an analysis chip that is a syringe-type chip, comprising:
a syringe main body having an openable and closable
tip;
a sample chamber comprising an openable and closable sample inlet port and being connected to a tip region of the syringe main body; and
a piston to be inserted into the syringe main body; and an analysis device into which the analysis chip is to be inserted, comprising:
a main body case that is a housing, the housing
comprising:
an insertion opening into which the syringe-type chip is to be inserted; and
a void in communication with the insertion opening, wherein
an opposite end of the insertion opening in an axial direction is a bottom;
a heating unit that heats the syringe-type chip;
a light source unit that emits light to the syringe-type chip; and
a detection unit disposed at the bottom of the housing, wherein
in use, the syringe-type chip is inserted into the analysis device.

11. The analysis kit according to claim 10, wherein
the syringe-type chip further comprises a negative pressure generator,
the negative pressure generator is connected to the tip region of the syringe main body, and
a connection site of the negative pressure generator and the tip region is located closer to the tip of the syringe main body in comparison with a connection site of the tip region and the sample chamber.

12. The analysis kit according to any one of claims 1 to 4, further satisfying the requirements of the analysis kit according to any one of claims 5 to 9.

13. The analysis kit according to claim 12, further satisfying the requirements of the analysis kit according to claim 10 or 11.

14. The analysis kit according to any one of claims 1 to 4, further satisfying the requirements of the analysis kit according to claim 10 or 11.

15. The analysis kit according to any one of claims 5 to 9, further satisfying the requirements of the analysis kit according to claim 10 or 11.

16. The analysis kit according to any one of claims 1 to 15, wherein
the heating unit and the light source unit are respectively disposed on the inner surfaces of the void along the axial direction.

17. The analysis kit according to claim 16, wherein
the heating unit and the light source unit each are disposed on each of the inner surfaces of the void facing each other.

18. The analysis kit according to claim 16 or 17, wherein
the analysis device further comprises a heat insulation section, and the heating unit is disposed on the inner surface of the void through the heat insulation section.

19. The analysis kit according to any one of claims 1 to 18, wherein
the detection unit is at least one of CCD and APD.

20. The analysis kit according to any one of claims 1 to 19, wherein the piston comprises an optical fiber.

21. The analysis kit according to any one of claims 1 to 20, wherein
the syringe-type chip comprises a condenser lens therein.

22. The analysis kit according to any one of claims 1 to 21, further comprising:
a power supply; or
a connector to be connected to an external power supply.

23. The analysis kit according to claim 22, wherein
the external power supply is a personal computer, a tablet, or a cellular phone.

24. The analysis kit according to claim 22, wherein
the connector is a USB.

25. The analysis kit according to any one of claims 1 to 24, wherein
the housing comprises a guide through which the syringe-type chip is introduced into the housing.

26. The analysis kit according to any one of claims 1 to 25, wherein
the analysis kit is for nucleic acid analysis.

27. The analysis kit according to any one of claims 1 to 26, wherein
the syringe-type chip comprises a reagent therein.

28. The analysis kit according to claim 27, wherein
the reagent comprises an analysis reagent that reacts with a target in a sample.

29. The analysis kit according to claim 28, wherein
the target is a nucleic acid, and
the analysis reagent comprises a reagent for nucleic acid amplification.

30. The analysis kit according to any one of claims 27 to 29, wherein
the target is a nucleic acid, and
the reagent comprises a reagent for nucleic acid detection.

31. The analysis kit according to claim 30, wherein
the reagent for nucleic acid detection is a fluorescence reagent.

32. The analysis kit according to any one of claims 27 to 31, wherein
the reagent comprises a pretreatment reagent of a sample.

33. The analysis kit according to any one of claims 1 to 32, wherein
the analysis chip is a connected syringe-type chip in which plural syringe-type chips are connected in parallel, and
the analysis device is a multi-analysis device comprising a plurality of detection units corresponding to the plural syringe-type chips.

34. The analysis kit according to claim 33, wherein
in a state where the connected syringe-type chip is inserted in the analysis device,
the heating unit is disposed on the inner surface of the void so as to face a parallel surface of each syringe-type chip of the connected syringe-type chip, and
the light source unit is disposed at least above or below the syringe-type chip at at least one end of the connected syringe-type chip and extended along the axial direction of the connected syringe-type chip.

35. The analysis kit according to claim 33 or 34, wherein
the light source unit comprises:
a linear light source; and
a converter that converts linear light into surface light,
wherein
the linear light source emits linear light along the axial direction of the connected syringe-type chip, and
the converter converts the linear light into surface light and emits the surface light.

36. The analysis kit according to any one of claims 33 to 35, wherein
the parallel surface of each syringe-type chip of the connected syringe-type chip is a translucent parallel surface, and
a side surface of each syringe-type chip that is in contact with an adjacent syringe-type chip in the connected syringe-type chip has light blocking ability.

37. The analysis kit according to any one of claims 33 to 36, wherein
at least two syringe-type chips of the connected syringe-type chip comprise analysis reagents that react with different targets in a sample.

38. An analysis method of a sample using the analysis kit according to any one of claims 1 to 37, comprising the following steps:
introducing a sample into the syringe-type chip;
mixing the sample and a reagent in the syringe-type chip;
inserting the syringe-type chip into the analysis device;
causing the sample to thermally react with the reagent in the syringe-type chip by the heating unit of the analysis device; and
detecting the reaction in the syringe-type chip by the detection unit of the analysis device.

39. The analysis method according to claim 38, wherein
the analysis kit is the analysis kit according to any one of claims 1 to 4, and
in the insertion step,
the syringe-type chip is inserted into the analysis device and the detection unit of the analysis device is inserted into the piston of the syringe-type chip.

40. The analysis method according to claim 38, wherein
the analysis kit is the analysis kit according to any one of claims 5 to 9, and
in the mixing step,
the sample and the reagent are mixed using the bead of the syringe-type chip.

41. The analysis method according to claim 40, wherein
the bead is a magnetic bead, and
in the insertion step,
the syringe-type chip is inserted into the analysis device, and the magnetic bead of the syringe-type chip is captured by the magnet of the analysis device.

42. The analysis method according to claim 40 or 41, wherein
after the insertion step and before the reaction step, a negative pressure is generated in the syringe main body by a negative pressure generator of the syringe-type chip.

43. The analysis method according to claim 38, wherein
the analysis kit is the analysis kit according to claim 10 or 11, and
in the introduction step,
after the sample has introduced into the sample chamber of the syringe-type chip through the sample inlet port, the sample is introduced into the syringe main body from the sample chamber.

44. The analysis method according to claim 43, wherein
after the introduction step and before the reaction step, a negative pressure is generated in the syringe main body by a negative pressure generator of the syringe-type chip.

45. The analysis method according to any one of claims 38 to 44, wherein
an analysis target is a nucleic acid.

46. The analysis method according to any one of claims 38 to 45, wherein
the sample is a biological sample.

47. The analysis method according to claim 46, wherein
the biological sample is at least one selected from the group consisting of blood, blood plasma, and blood serum.

48. The analysis method according to any one of claims 38 to 47, wherein
an analysis target is a virus or a bacterium.
